# EUROPEAN PATENT SPECIFICATION

(11) **EP 0 974 604 B1**
(45) Date of publication and mention of the grant of the patent: **07.05.2003**
(21) Application number: 99121733.2
(22) Date of filing: 17.03.1994
(51) Int. Cl.: C07C 17/087, C08F 14/06, C08F 14/18, C07C 17/00

(54) **Method of synthesising 1-halo-1-iodoethane**
Verfahren zur Herstellung von 1-Halogen-1-jodethan
Procédé de préparation de 1-halo-1-iodoéthane

(30) Priority: 22.03.1993 US 35017; 22.03.1993 US 34981
(43) Date of publication of application: 26.01.2000
(62) Divisional of application: 94104143.6
(73) Proprietor: Oxy Vinyls L.P., Dallas, Texas 75244 (US)
(72) Inventor: Bak, Philip I., Amherst, Ohio 44001 (US); Bidinger, Gregory, P., Akron, Ohio 44321 (US); Gozens, Ross J., Strongsville, Ohio 44136 (US); Klich, Paul R., Lyndhurst, Ohio 44124 (US); Mayer, Lance A., Strongsville, Ohio 44136 (US)
(74) Representative: Weber, Thomas, Dr.

(56) References cited:
- KHARASCH ET AL.: "The peroxide Effect in the Addition of Reactants To Unasturated Compounds IV. The Addition of Halogen Acids to Vinyl Chloride" J.AMERICAN CHEM. SOC., vol. 56, no. 1, 1934, pages 712-714, XP002141721

## Description

### BACKGROUND OF THE INVENTION

### Field of Invention

The present invention relates to a simple, efficient and reliable method for synthesizing 1-halo-1-iodoethane by reacting hydrogen iodide with vinyl halide in the presence of an iodine generating catalyst.

### Description of the Art

The addition of hydrogen iodide to vinyl chloride was first described by Kharasch and Hannum in an article in 1934. The article, entitled "The Peroxide Effect in the Addition of Reagents to Unsaturated Compounds IV. The Addition of Halogen Acids to Vinyl Chloride", J. American Chem. Soc., 56, (1934) p. 712 described the addition of various halogen acids such as hydrogen iodide to vinyl chloride. In particular, Kharasch *et. al*. discuss the addition of 0.12 moles of hydrogen iodide to 0.1 mole of vinyl chloride in a reactor. Kharasch *et. al.* were studying the effects of peroxides on reactions which occur via a carbonation process in contrast to the present invention for free radical processes.

An attempt was made to reproduce the work of Kharasch and Hannum using pure reagents. Using the Kharasch procedure, there was no 1-chloro-1-iodoethane synthesized. However, applicants have discovered a novel process for synthesizing 1-chloro-1-iodoethane. The 1-chloro-1-iodoethane is useful as an iodine containing chain transfer agent in the polymerization of vinyl chloride, resulting in a vinyl chloride polymer having good thermal stability along with low molecular weight and low polydispersity.

In accordance with the present invention there is provided a method for synthesizing 1-halo-1-iodoethane, by reacting hydrogen iodide with vinyl halide in the presence of an iodine generating catalyst.

The resulting product is formed in high yield, and high purity 1-halo-1-iodoethane is obtained.

The present invention pertains to the synthesis of 1-halo-1-iodoethane. The synthesis can occur in any of two possible ways. First, the 1-halo-1-iodoethane can be synthesized separately, purified and used as desired. Alternatively, 1-halo-1-iodoethane can be synthesized in the reaction vessel prior to the polymerization of vinyl halide monomer. This synthesis pertains to vinyl chloride or vinyl fluoride in-situ the addition of the appropriate quantity of hydrogen iodide and iodine catalyst is combined with a portion of vinyl halide monomer charged to the reaction vessel. Prior to initiation, molecular iodine liberated from the catalyst is preferably reduced by a suitable reducing agent and care must be taken to eliminate I₂ prior to polymerization.

The starting reagent for making 1-chloro-1-iodoethane of this invention is vinyl chloride. Correspondingly, the starting reagent for synthesizing 1-fluoro-1-iodoethane is vinyl fluoride. For the sake of brevity the synthesis will be described using vinyl chloride, but is equally adaptable with vinyl fluoride. Preferably, the vinyl chloride should be of high purity and dryness. Moreover, a slight excess of vinyl chloride monomer over hydrogen iodide is recommended to ensure complete consumption of substantially anhydrous hydrogen iodide.

The hydrogen iodide is added to vinyl chloride monomer in a reactor in the presence of an iodine catalyst. The iodine catalyst can be any iodine liberating compound and which will not in itself react with vinyl chloride or hydrogen iodide. The iodine catalyst can be an organic iodide, inorganic iodide or molecular iodine. Specific examples include KI, NaI, LiI, BrI, ClI, FI, and alkyl iodides such as CH₃CH₂CHI₂ and CHI₃. The most preferred iodine catalyst is molecular iodine. Generally, 0.1 to 2 mole % catalyst is used. Preferably, 0.1 to 1 mole % is used.

The synthesis is carried out in any reaction vessel suitable for the reaction of vinyl chloride monomer and is within the knowledge of one of ordinary skill in the art. The reaction temperature is about -75°C to 100°C. Most preferably, the reaction temperature is about -50°C to -45°C. The components react for approximately one to five hours in the reactor. Completion is indicated when the reactor pressure drops to the vapor pressure of the vinyl chloride. Using the instant method under specified conditions renders a yield in excess of 80 percent. The resulting 1-chloro-1-iodoethane may be purified as described below.

Iodine, which hinders vinyl halide monomer polymerization, should be removed from the 1-chloro-1-iodoethane which is formed. A suitable reducing agent such as sodium thiosulfate in aqueous solution can be mixed with the crude product followed by removal of the aqueous layer. If an organic iodide is used as the catalyst for the reaction between vinyl halide and hydrogen iodide, then the reaction product should be purified by distillation. If molecular iodine or an inorganic iodide is used as catalyst for the reaction between vinyl halide and hydrogen iodide, then the 1-chloro-1-iodoethane may be purified simply by washing with sodium thiosulfate, followed by washing with water and finally drying the product over an anhydrous drying agent, e.g., magnesium sulfate. It is suggested that the 1-chloro-1-iodoethane should be stored in the dark over copper turnings to inhibit degradation.

### EXAMPLES

### Example 1 - Synthesis of 1-chloro-1-iodoethane

A 1-liter three-necked flask, equipped with nitrogen ("N₂") purge, stirrer, and cold finger condenser was assembled. The flask was immersed in a dry ice/acetone bath and the cold finger filled with the same. The flask was flushed with N₂. 294.3 grams vinyl chloride monomer ("VCM") (4.71 moles) were slowly added from a vessel via Teflon tubing to the flask. The VCM condensed upon contact with the cooled flask walls. 105.2 grams hydrogen iodide ("HI") (0.82 moles) were slowly bubbled through the VCM via Teflon tubing. Then, approximately 3 grams (0.01 moles) iodine crystals were added. The contents of the flask were stirred at dry ice/acetone temperature for three hours and then the bath was removed. The cold finger was maintained for an additional two hours. Excess of the reagents were permitted to evaporate as the reaction mixture warmed to room temperature. The were 131.0 grams of the product isolated as a purple liquid. The yield of 1-chloro-1-iodoethane was about 83.65%.

The purple liquid was found to lose its color in the presence of sodium thiosulfate. All of the liquid was added to a separatory funnel. An equal amount of 0.1 N sodium thiosulfate was added. With continued shaking, a pale yellow organic layer (bottom layer) was obtained. The organic liquid was dried over magnesium sulfate.

Upon standing, the filtered material began to turn pink. Copper filings were added and two days later, the product was found to be a very pale yellow. A week later the color had not changed.

A 13-CNMR spectrum which exhibits signals at 22.08 and 35.07 ppm and trace impurity signals at approximately 85 and 130 ppm is consistent with the structure for 1-chloro-1-iodoethane.

### Comparative Example 2

The reaction was carried out in a 1 liter glass pressure vessel, equipped with an agitator according to the method of Kharasch and Hannum discussed above. The vessel was placed under an atmosphere of nitrogen. When the vessel was cooled to around 0°C, vinyl chloride monomer (125 grams, 2 moles) was added. With the temperature held at or below 0°C, approximately 245 grams (1.9 moles) of HI vapor were transferred into the vinyl chloride using a Teflon line. The colorless mixture of liquid was then stirred and the temperature allowed to warm to room temperature over a period of approximately four hours. During that time, the reaction mixture became a pale pink in color. On subsequent evaporation of excess reagents, no measurable yield of 1-chloro-1-iodoethane was obtained.

The fact that no 1-chloro-1-iodoethane was obtained using the Kharasch *et. al.* method reaffirms that Applicants' iodine containing catalyst is necessary to render 1-chloro-1-iodoethane.

## Claims

1. A method for synthesizing 1-halo-1-iodoethane, by reacting hydrogen iodide with vinyl halide in the presence of an iodine generating catalyst.

2. The method of claim 1 wherein said vinyl halide is vinyl chloride and 1-halo-1-iodoethane is 1-chloro-1-iodoethane.

3. The method of claim 1 wherein said iodine generating catalyst is an organic iodide.

4. The method of claim 1 wherein said iodine generating catalyst is molecular iodine.

5. The method of claim 1 wherein said iodine containing catalyst is an inorganic iodide.

6. The method of claim 3 wherein said catalyst is an alkyl iodide.

7. The method of claim 5 wherein said catalyst selected from the group consisting of KI, NaI, LiI, BrI, ClI, and FI.

## Patentansprüche

1. Verfahren zur Herstellung von 1-Halogen-1-iodethan durch Umsetzung von Iodwasserstoff mit Vinylhalogenid in Gegenwart eines Ioderzeugenden Katalysators.

2. Verfahren gemäß Anspruch 1, wobei das Vinylhalogenid Vinylchlorid ist und das 1-Halogen-1-iodethan 1-Chlor-1-iodethan ist.

3. Verfahren gemäß Anspruch 1, wobei der Iod-erzeugende Katalysator ein organisches Iodid ist.

4. Verfahren gemäß Anspruch 1, wobei der Iod-erzeugende Katalysator molekulares Iod ist.

5. Verfahren gemäß Anspruch 1, wobei der Iod-enthaltende Katalysator ein anorganisches Iodid ist.

6. Verfahren gemäß Anspruch 3, wobei der Katalysator ein Alkyliodid ist.

7. Verfahren gemäß Anspruch 5, wobei der Katalysator aus der aus KI, NaI, LiI, BrI, CII und FI bestehenden Gruppe ausgewählt ist.

## Revendications

1. Procédé pour synthétiser un 1-halo-1-iodoéthane, en faisant réagir de l'iodure d'hydrogène avec de l'halogénure vinylique en présence d'un catalyseur de génération d'iode.

2. Procédé selon la revendication 1, dans lequel ledit halogénure vinylique est le chlorure de vinyle et le 1-halo-1-iodoéthane est le 1-chloro-1-iodoéthane.

3. Procédé selon la revendication 1, dans lequel ledit catalyseur de génération d'iode est un iodure organique.

4. Procédé selon la revendication 1, dans lequel ledit catalyseur de génération d'iode est de l'iode moléculaire.

5. Procédé selon la revendication 1, dans lequel ledit catalyseur contenant l'iode est un iodure inorganique.

6. Procédé selon la revendication 3, dans lequel ledit catalyseur est un iodure d'alkyle.

7. Procédé selon la revendication 5, dans lequel ledit catalyseur est choisi dans le groupe qui est constitué de KI, NaI, LiI, BrI, ClI et FI.
